# EUROPEAN PATENT SPECIFICATION

(11) **EP 3 546 572 B1**
(45) Date of publication and mention of the grant of the patent: **13.03.2024**
(21) Application number: 19161861.0
(22) Date of filing: 12.05.2014
(51) Int. Cl.: C12N 9/22, C12N 15/63, C07K 16/28, C07K 19/00, C07K 14/725, A61K 38/00, A61K 35/17, C07K 14/705, C12N 5/0783, A61P 35/00, A61P 35/02

(54) **CD19 SPECIFIC CHIMERIC ANTIGEN RECEPTOR AND USES THEREOF**
CHIMÄRER MEHRSTRÄNGIGER CD19-ANTIGEN-REZEPTOR UND VERWENDUNGEN DAVON
RÉCEPTEUR DE L'ANTIGÈNE CHIMÈRE SPÉCIFIQUE DE CD19 ET SES UTILISATIONS

(30) Priority: 13.05.2013 WO PCT/US2013/040755; 13.05.2013 US 201313892805; 13.05.2013 WO PCT/US2013/040766; 08.10.2013 US 201361888259 P
(43) Date of publication of application: 02.10.2019
(62) Divisional of application: 14723436.3
(73) Proprietor: Cellectis, 75013 Paris (FR)
(72) Inventor: SCHIFFER-MANNIOUI, Cècile, 94350 Villiers-sur-Marne (FR); GALETTO, Roman, 75014 Paris (FR); SMITH, Julianne, New York, NY 10128 (US); SCHARENBERG, Andrew, Seattle, WA 98177 (US)
(74) Representative: Zacco Denmark A/S

(56) References cited:
- KEICHIRO MIHARA ET AL: "Synergistic and persistent effect of T-cell immunotherapy with anti-CD19 or anti-CD38 chimeric receptor in conjunction with rituximab on B-cell non-Hodgkin lymphoma", BRITISH JOURNAL OF HAEMATOLOGY, vol. 151, no. 1, 1 October 2010 (2010-10-01), pages 37-46, XP055086001, ISSN: 0007-1048, DOI: 10.1111/j.1365-2141.2010.08297.x
- LESLIE E HUYE ET AL: "Combining mTor Inhibitors With Rapamycin-resistant T Cells: A Two-pronged Approach to Tumor Elimination", MOLECULAR THERAPY, vol. 19, no. 12, 30 December 2011 (2011-12-30), pages 2239-2248, XP055223219, ISSN: 1525-0016, DOI: 10.1038/mt.2011.179
- HELEN E HESLOP: "Safer CARS", MOLECULAR THERAPY, vol. 18, no. 4, 1 April 2010 (2010-04-01), pages 661-662, XP55609534, ISSN: 1525-0016, DOI: 10.1038/mt.2010.42
- LAURENT POIROT ET AL: "Multiplex Genome-Edited T-cell Manufacturing Platform for "Off-the-Shelf" Adoptive T-cell Immunotherapies", CANCER RESEARCH, vol. 75, no. 18, 16 July 2015 (2015-07-16) , pages 3853-3864, XP055568648, US ISSN: 0008-5472, DOI: 10.1158/0008-5472.CAN-14-3321
- L. J. N. Cooper ET AL: "Good T cells for bad B cells", Blood, vol. 119, no. 12, 22 March 2012 (2012-03-22), pages 2700-2702, XP055338012, US ISSN: 0006-4971, DOI: 10.1182/blood-2011-12-398719

## Description

### Field of the invention

The present invention relates to a composition for use in combination therapy comprising a) an engineered T-cell expressing a CD19-specific Chimeric Antigen Receptor which comprises at least one extracellular ligand binding domain specific for CD19, a transmembrane domain, and at least one intracellular signaling domain; wherein said extracellular domain comprises a single chain FV fragment derived from the monoclonal antibody 4G7, specific for CD19, said single chain FV fragment comprising the variable fragment of the CD19 monoclonal antibody 4G7 immunoglobulin gamma 1 heavy chain of SEQ ID NO: 3 and the variable fragment of the CD19 monoclonal antibody 4G7 immunoglobulin kappa light chain of SEQ ID NO: 4 or SEQ ID NO: 5; and b) the antibody alemtuzumab. The present invention is defined in the appended claims.

### Background of the invention

Adoptive immunotherapy, which involves the transfer of autologous antigen-specific T cells generated *ex vivo,* is a promising strategy to treat viral infections and cancer. The T cells used for adoptive immunotherapy can be generated either by expansion of antigen-specific T cells or redirection of T cells through genetic engineering (Park, Rosenberg et al. 2011). Transfer of viral antigen specific T cells is a well-established procedure used for the treatment of transplant associated viral infections and rare viral-related malignancies. Similarly, isolation and transfer of tumor specific T cells has been shown to be successful in treating melanoma.

Novel specificities in T cells have been successfully generated through the genetic transfer of transgenic T cell receptors or chimeric antigen receptors (CARs) (Jena, Dotti et al. 2010). CARs are synthetic receptors consisting of a targeting moiety that is associated with one or more signaling domains in a single fusion molecule. In general, the binding moiety of a CAR consists of an antigen-binding domain of a single-chain antibody (scFv), comprising the light and variable fragments of a monoclonal antibody joined by a flexible linker. Binding moieties based on receptor or ligand domains have also been used successfully. The signaling domains for first generation CARs are derived from the cytoplasmic region of the CD3zeta or the Fc receptor gamma chains. First generation CARs have been shown to successfully redirect T cell cytotoxicity, however, they failed to provide prolonged expansion and anti-tumor activity in *vivo.* Signaling domains from co-stimulatory molecules including CD28, OX-40 (CD134), and 4-1BB (CD137) have been added alone (second generation) or in combination (third generation) to enhance survival and increase proliferation of CAR modified T cells. CARs have successfully allowed T cells to be redirected against antigens expressed at the surface of tumor cells from various malignancies including lymphomas and solid tumors (Jena, Dotti et al. 2010).

CD19 is an attractive target for immunotherapy because the vast majority of B-acute lymphoblastic leukemia (B-ALL) uniformly express CD19, whereas expression is absent on non hematopoietic cells, as well as myeloid, erythroid, and T cells, and bone marrow stem cells. Clinical trials targeting CD19 on B-cell malignancies are underway with encouraging anti-tumor responses. Most infuse T cells genetically modified to express a chimeric antigen receptor (CAR) with specificity derived from the scFv region of a CD19-specific mouse monoclonal antibody FMC63 (Nicholson, Lenton et al. 1997; Cooper, Topp et al. 2003; Cooper, Jena et al. 2012) (International application: WO2013/126712). For example, Cooper, Jena et al. 2012 (Blood, vol. 119, no. 12, pages 2700-2702) provide a review of the published clinical data on T cells modified to express a chimeric antigen receptor (CAR) with specificity derived from the scFv region of the CD19-specific mouse monoclonal antibody FMC63.

However, there is still a need to improve construction of CARs that show better compatibility with T-cell proliferation, in order to allow the cells expressing such CARs to reach significant clinical advantage.

### Summary of the invention

The inventors have generated a CD19 specific CAR (4G7-CAR) comprising a scFV derived from the CD19 specific monoclonal antibody 4G7, and have surprisingly found that introduction of the resulting 4G7-CAR into primary T cells could confer a prolonged "activated" state on the transduced cell independently of antigen binding. Following non-specific activation in vitro (e.g. with anti CD3/CD28 coated beads and recombinant IL2), these cells displayed an increased cell size (blast formation) as well as the expression of activation markers (CD25) over an extended time period compared to cells transduced with a similar CAR comprising the FMC63 scFV. This long-term activation permits extended proliferation and provides an antigen-independent mechanism for expansion of 4G7-CAR cells in vitro.

The present invention thus provides a composition for use in combination therapy comprising a) an engineered T-cell expressing a chimeric antigen receptor comprising at least one extracellular ligand binding domain, a transmembrane domain and at least one signal transducing domain, wherein said extracellular ligand binding domain comprises a scFV derived from specific monoclonal antibody 4G7, said single chain FV fragment comprising the variable fragment of the CD19 monoclonal antibody 4G7 immunoglobulin gamma 1 heavy chain of SEQ ID NO: 3 and the variable fragment of the CD19 monoclonal antibody 4G7 immunoglobulin kappa light chain of SEQ ID NO: 4 or SEQ ID NO: 5; and b) the antibody alemtuzumab. In particular, the CAR employed in accordance with the present invention once transduced into a T cell contributes to antigen independent activation and proliferation of the cell.

### Brief description of the figures

**Figure 1****:** Proliferation ofTCR alpha inactivated T cells (KO) transduced with 4G7-CAR lentiviral vector compared to non transduced KO T cells (NTD). Proliferation was followed during 30 days after (IL2+CD28) or not (IL2) a step of reactivation with soluble anti-CD28.
**Figure 2****:** CD25 activation marker expression analysis at the surface of inactivated TCR alpha T cells transduced with 4G7-CAR lentiviral vector, gated on the basis of 4G7-CAR expression (CAR+, CAR-) and compared to CD25 expression on TCR alpha positive non electroporated (NEP) or TCR alpha disrupted but non tranduced (NTD) cells. CD25 expression was analyzed after (IL2+CD28) or not (IL2) a step of reactivation with soluble anti-CD28.
**Figure 3**: CAR expression analysis at the surface of T cells transduced with a lentiviral vector encoding either the 4G7-CAR or the FMC63-CAR. The analysis was done 3, 8 and 15 days post transduction by flow cytometry. NT refers to no transduced T cells.
**Figure 4****:** CD25 expression analysis at the surface of T cells transduced with a lentiviral vector encoding either the 4G7-CAR or the FMC63-CAR. The analysis was done 3, 8 and 15 days post transduction by flow cytometry. NT refers to no transduced T cells.
**Figure 5****:** Size analysis of T cells transduced with a lentiviral vector encoding either the 4G7-CAR or the FMC63-CAR. The analysis was done 3, 8 and 15 days post transduction by flow cytometry. NT refers to no transduced T cells.
**Figure 6****:** Proliferation of T cells transduced with 4G7-CAR compared to FMC63 lentiviral vector. Proliferation was followed during 20 days after (CD28) or not (-) a step of reactivation with soluble anti-CD28. NTD refers to no transduced T cells.

### Detailed description of the invention

Unless specifically defined herein, all technical and scientific terms used have the same meaning as commonly understood by a skilled artisan in the fields of gene therapy, biochemistry, genetics, and molecular biology.

All methods and materials similar or equivalent to those described herein can be used in the practice or testing of the present invention, with suitable methods and materials being described herein. In case of conflict, the present specification, including definitions, will prevail. Further, the materials, methods, and examples are illustrative only and are not intended to be limiting, unless otherwise specified.

The practice of the present invention will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Current Protocols in Molecular Biology (Frederick M. AUSUBEL, 2000, Wiley and son Inc, Library of Congress, USA); Molecular Cloning: A Laboratory Manual, Third Edition, (Sambrook et al, 2001, Cold Spring Harbor, New York: Cold Spring Harbor Laboratory Press); Oligonucleotide Synthesis (M. J. Gait ed., 1984); Mullis et al. U.S. Pat. No. 4,683,195; Nucleic Acid Hybridization (B. D. Harries & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the series, Methods In ENZYMOLOGY (J. Abelson and M. Simon, eds.-in-chief, Academic Press, Inc., New York), specifically, Vols.154 and 155 (Wu et al. eds.) and Vol. 185, "Gene Expression Technology" (D. Goeddel, ed.); Gene Transfer Vectors For Mammalian Cells (J. H. Miller and M. P. Calos eds., 1987, Cold Spring Harbor Laboratory); Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986); and Manipulating the Mouse Embryo, (Cold Spring Harbor Laboratory Press, Cold Spring Harbor, N.Y., 1986).

### CD19 specific Chimeric Antigen Receptor

The present invention relates to a composition for use in combination therapy comprising a) a T-cell expressing a chimeric antigen receptor (CAR) comprising an extracellular ligand-binding domain, a transmembrane domain and a signaling transducing domain, wherein said extracellular domain comprises a single chain FV fragment derived from the monoclonal antibody 4G7, specific for CD19, said single chain FV fragment comprising the variable fragment of the CD19 monoclonal antibody 4G7 immunoglobulin gamma 1 heavy chain of SEQ ID NO: 3 and the variable fragment of the CD19 monoclonal antibody 4G7 immunoglobulin kappa light chain of SEQ ID NO: 4 or SEQ ID NO: 5; and b) the antibody alemtuzumab.

The term "extracellular ligand-binding domain" as used herein is defined as an oligo- or polypeptide that is capable of binding a ligand. Preferably, the domain will be capable of interacting with a cell surface molecule. For example, the extracellular ligand-binding domain may be chosen to recognize a ligand that acts as a cell surface marker on target cells associated with a particular disease state.

In a preferred embodiment, said extracellular ligand-binding domain comprises a single chain antibody fragment (scFv) comprising the variable fragments of the CD19 monoclonal antibody 4G7 immunoglobulin gamma 1 heavy chain of SEQ ID NO: 3 and the variable fragments of the CD19 monoclonal antibody 4G7 immunoglobulin kappa light chain of SEQ ID NO: 4 or SEQ ID NO: 5 linked together by a flexible linker. In particular embodiment said flexible linker has the amino acid sequence of SEQ ID NO: 6.

In other words, said CAR comprises an extracellular ligand-biding domain which comprises a single chain FV fragment derived from the CD19 specific monoclonal antibody 4G7 (Peipp, Saul et al. 2004). In a particular embodiment, said scFV comprises the amino acid sequence of SEQ ID NO: 7 or SEQ ID NO: 8.

The signal transducing domain or intracellular signaling domain of the CAR according to the present invention is responsible for intracellular signaling following the binding of extracellular ligand binding domain to the target resulting in the activation of the immune cell and immune response. In other words, the signal transducing domain is responsible for the activation of at least one of the normal effector functions of the immune cell in which the CAR is expressed. For example, the effector function of a T cell can be a cytolytic activity or helper activity including the secretion of cytokines. Thus, the term "signal tansducing domain" refers to the portion of a protein which transduces the effector signal function signal and directs the cell to perform a specialized function.

Preferred examples of signal transducing domain for use in a CAR can be the cytoplasmic sequences of the T cell receptor and co-receptors that act in concert to initiate signal transduction following antigen receptor engagement, as well as any derivate or variant of these sequences and any synthetic sequence that has the same functional capability. Signal transduction domain comprises two distinct classes of cytoplasmic signaling sequence, those that initiate antigen-dependent primary activation, and those that act in an antigen-independent manner to provide a secondary or co-stimulatory signal. Primary cytoplasmic signaling sequence can comprise signaling motifs which are known as immunoreceptor tyrosine-based activation motifs of ITAMs. ITAMs are well defined signaling motifs found in the intracytoplasmic tail of a variety of receptors that serve as binding sites for syk/zap70 class tyrosine kinases. Examples of ITAM used in the invention can include as non limiting examples those derived from TCRzeta, FcRgamma, FcRbeta, FcRepsilon, CD3gamma, CD3delta, CD3epsilon, CD5, CD22, CD79a, CD79b and CD66d. In a preferred embodiment, the signaling transducing domain of the CAR can comprise the CD3zeta signaling domain which has amino acid sequence with at least 70%, preferably at least 80%, more preferably at least 90 %, 95 % 97 % or 99 % sequence identity with amino acid sequence selected from the group consisting of SEQ ID NO: 10.

In particular embodiment the signal transduction domain of the CAR of the present invention comprises a co-stimulatory signal molecule. A co-stimulatory molecule is a cell surface molecule other than an antigen receptor or their ligands that is required for an efficient immune response. "Co-stimulatory ligand" refers to a molecule on an antigen presenting cell that specifically binds a cognate co-stimulatory molecule on a T-cell, thereby providing a signal which, in addition to the primary signal provided by, for instance, binding of a TCR/CD3 complex with an MHC molecule loaded with peptide, mediates a T cell response, including, but not limited to, proliferation activation, differentiation and the like. A co-stimulatory ligand can include but is not limited to CD7, B7-1 (CD80), B7-2 (CD86), PD-L1, PD-L2, 4-1BBL, OX40L, inducible costimulatory igand (ICOS-L), intercellular adhesion molecule (ICAM, CD30L, CD40, CD70, CD83, HLA-G, MICA, M1CB, HVEM, lymphotoxin beta receptor, 3/TR6, ILT3, ILT4, an agonist or antibody that binds Toll ligand receptor and a ligand that specifically binds with B7-H3. A co-stimulatory ligand also encompasses, inter alia, an antibody that specifically binds with a co-stimulatory molecule present on a T cell, such as but not limited to, CD27, CD28, 4-1BB, OX40, CD30, CD40, PD-1, ICOS, lymphocyte function-associated antigen-1 (LFA-1), CD2, CD7, LTGHT, NKG2C, B7-H3, a ligand that specifically binds with CD83.

A "co-stimulatory molecule" refers to the cognate binding partner on a T-cell that specifically binds with a co-stimulatory ligand, thereby mediating a co-stimulatory response by the cell, such as, but not limited to proliferation. Co-stimulatory molecules include, but are not limited to an MHC class I molecule, BTLA and Toll ligand receptor. Examples of costimulatory molecules include CD27, CD28, CD8, 4-1BB (CD137), OX40, CD30, CD40, PD-1, ICOS, lymphocyte function-associated antigen-1 (LFA-1), CD2, CD7, LIGHT, NKG2C, B7-H3 and a ligand that specifically binds with CD83 and the like.

In a preferred embodiment, the signal transduction domain of the CAR of the present invention comprises a part of co-stimulatory signal molecule selected from the group consisting of fragment of 4-1BB (GenBank: AAA53133.) and CD28 (NP_006130.1). In particular the signal transduction domain of the CAR of the present invention comprises amino acid sequence which comprises at least 70%, preferably at least 80%, more preferably at least 90 %, 95 % 97 % or 99 % sequence identity with amino acid sequence selected from the group consisting of SEQ ID NO: 11 and SEQ ID NO: 12.

The CAR according to the present invention is expressed on the surface membrane of the cell. Thus, the CAR can comprise a transmembrane domain. The distinguishing features of appropriate transmembrane domains comprise the ability to be expressed at the surface of a cell, preferably in the present invention an immune cell, in particular lymphocyte cells or Natural killer (NK) cells, and to interact together for directing cellular response of immune cell against a predefined target cell. The transmembrane domain can be derived either from a natural or from a synthetic source. The transmembrane domain can be derived from any membrane-bound or transmembrane protein. As non limiting examples, the transmembrane polypeptide can be a subunit of the T cell receptor such as α, β, γ or δ, polypeptide constituting CD3 complex, IL2 receptor p55 (α chain), p75 (β chain) or γ chain, subunit chain of Fc receptors, in particular Fcy receptor III or CD proteins. Alternatively the transmembrane domain can be synthetic and can comprise predominantly hydrophobic residues such as leucine and valine. In a preferred embodiment said transmembrane domain is derived from the human CD8 alpha chain (e.g. NP_001139345.1). The transmembrane domain can further comprise a stalk region between said extracellular ligand-binding domain and said transmembrane domain. The term "stalk region" used herein generally means any oligo- or polypeptide that functions to link the transmembrane domain to the extracellular ligand-binding domain. In particular, stalk region are used to provide more flexibility and accessibility for the extracellular ligand-binding domain. A stalk region may comprise up to 300 amino acids, preferably 10 to 100 amino acids and most preferably 25 to 50 amino acids. Stalk region may be derived from all or part of naturally occurring molecules, such as from all or part of the extracellular region of CD8, CD4 or CD28, or from all or part of an antibody constant region. Alternatively the stalk region may be a synthetic sequence that corresponds to a naturally occurring stalk sequence, or may be an entirely synthetic stalk sequence. In a preferred embodiment said stalk region is a part of human CD8 alpha chain (e.g. NP_001139345.1). In another particular embodiment, said transmembrane and hinge domains comprise a part of human CD8 alpha chain, preferably which comprises at least 70%, preferably at least 80%, more preferably at least 90 %, 95 % 97 % or 99 % sequence identity with amino acid sequence selected from the group consisting of SEQ ID NO: 13.

In a particular embodiment, said Chimeric Antigen Receptor comprises a scFV comprising the variable fragment of the CD19 monoclonal antibody 4G7 immunoglobulin gamma 1 heavy chain of SEQ ID NO: 3 and the variable fragment of the CD19 monoclonal antibody 4G7 immunoglobulin kappa light chain of SEQ ID NO: 4 or SEQ ID NO: 5, a CD8 alpha human hinge and transmembrane domain, the CD3 zeta signaling domain and 4-1BB signaling domain. Preferably, the 4G7 CAR of the present invention comprises at least 70%, preferably at least 80%, more preferably at least 90 %, 95 % 97 % or 99 % sequence identity with amino acid sequence selected from the group consisting of SEQ ID NO: 14 and 15.

Downregulation or mutation of target antigens is commonly observed in cancer cells, creating antigen-loss escape variants. Thus, to offset tumor escape and render immune cell more specific to target, the CD19 specific CAR can comprise another extracellular ligand-binding domains, to simultaneously bind different elements in target thereby augmenting immune cell activation and function. In one embodiment, the extracellular ligand-binding domains can be placed in tandem on the same transmembrane polypeptide, and optionally can be separated by a linker. In another embodiment, said different extracellular ligand-binding domains can be placed on different transmembrane polypeptides composing the CAR. In another embodiment, the present invention relates to a population of CARs comprising each one different extracellular ligand binding domains. By population of CARs, it is meant at least two, three, four, five, six or more CARs each one comprising different extracellular ligand binding domains. The different extracellular ligand binding domains can preferably simultaneously bind different elements in target thereby augmenting immune cell activation and function.

### Polynucleotides, vectors:

Described are polynucleotides, vectors encoding the above described CAR. In a preferred instance, the a polynucleotide comprises the nucleic acid sequence SEQ ID NO: 17. In a preferred instance, the polynucleotide has at least 70%, preferably at least 80%, more preferably at least 90 %, 95 % 97 % or 99 % sequence identity with nucleic acid sequence selected from the group consisting of SEQ ID NO: 17.

The polynucleotide may consist in an expression cassette or expression vector (e.g. a plasmid for introduction into a bacterial host cell, or a viral vector such as a baculovirus vector for transfection of an insect host cell, or a plasmid or viral vector such as a lentivirus for transfection of a mammalian host cell).

In a particular instance, the different nucleic acid sequences can be included in one polynucleotide or vector which comprises a nucleic acid sequence encoding ribosomal skip sequence such as a sequence encoding a 2A peptide. 2A peptides, which were identified in the Aphthovirus subgroup of picornaviruses, causes a ribosomal "skip" from one codon to the next without the formation of a peptide bond between the two amino acids encoded by the codons (see (Donnelly and Elliott 2001; Atkins, Wills et al. 2007; Doronina, Wu et al. 2008)). By "codon" is meant three nucleotides on an mRNA (or on the sense strand of a DNA molecule) that are translated by a ribosome into one amino acid residue. Thus, two polypeptides can be synthesized from a single, contiguous open reading frame within an mRNA when the polypeptides are separated by a 2A oligopeptide sequence that is in frame. Such ribosomal skip mechanisms are well known in the art and are known to be used by several vectors for the expression of several proteins encoded by a single messenger RNA.

To direct, transmembrane polypeptide into the secretory pathway of a host cell, a secretory signal sequence (also known as a leader sequence, prepro sequence or pre sequence) is provided in polynucleotide sequence or vector sequence. The secretory signal sequence is operably linked to the transmembrane nucleic acid sequence, i.e., the two sequences are joined in the correct reading frame and positioned to direct the newly synthesized polypeptide into the secretory pathway of the host cell. Secretory signal sequences are commonly positioned 5' to the nucleic acid sequence encoding the polypeptide of interest, although certain secretory signal sequences may be positioned elsewhere in the nucleic acid sequence of interest (see, e.g., Welch et al., U.S. Patent No. 5,037,743; Holland et al., U.S. Patent No. 5,143,830). In a preferred instance the signal peptide comprises the amino acid sequence SEQ ID NO: 18 and 19.

Those skilled in the art will recognize that, in view of the degeneracy of the genetic code, considerable sequence variation is possible among these polynucleotide molecules. Preferably, the nucleic acid sequences disclosed herein are codon-optimized for expression in mammalian cells, preferably for expression in human cells. Codon-optimization refers to the exchange in a sequence of interest of codons that are generally rare in highly expressed genes of a given species by codons that are generally frequent in highly expressed genes of such species, such codons encoding the amino acids as the codons that are being exchanged.

In a preferred instance, the polynucleotide comprises the nucleic acid sequence selected from the group consisting of: SEQ ID NO: 17. Also described are polynucleotides comprising a nucleic acid sequence that has at least 70%, preferably at least 80%, more preferably at least 90 %, 95 % 97 % or 99 % sequence identity with nucleic acid sequence selected from the group consisting of SEQ ID NO: 17.

### Methods of engineering an immune cell:

Described is a method of preparing T cells for immunotherapy comprising introducing into said T cells the CAR as described above and expanding said cells. In particular, a method of engineering a T cell is described comprising providing a T cell and expressing at the surface of said cell at least one CAR as described above. In particular instance, the method comprises transforming the cell with at least one polynucleotide encoding CAR as described above, and expressing said polynucleotides into said cell.

In a preferred instance, said polynucleotides are included in lentiviral vectors in view of being stably expressed in the cells.

In another instance, said method further comprises a step of genetically modifying said cell by inactivating at least one gene expressing one component of the TCR, a target for an immunosuppressive agent, HLA gene and/or an immune checkpoint gene such as PDCD1 or CTLA-4. In a preferred instance, said gene is selected from the group consisting of TCRalpha, TCRbeta, CD52, GR, PD1 and CTLA-4. In a preferred instance said method further comprises introducing into said T cells a rare-cutting endonuclease able to selectively inactivate by DNA cleavage said genes. In a more preferred instance said rare-cutting endonuclease is TALE-nuclease or Cas9 endonuclease.

### Delivery methods

The different methods described above involve introducing CAR into a cell. As non-limiting example, said CAR can be introduced as transgenes encoded by one plasmidic vector. Said plasmid vector can also contain a selection marker which provides for identification and/or selection of cells which received said vector.

Polypeptides may be synthesized *in situ* in the cell as a result of the introduction of polynucleotides encoding said polypeptides into the cell. Alternatively, said polypeptides could be produced outside the cell and then introduced thereto. Methods for introducing a polynucleotide construct into cells are known in the art and including as non limiting examples stable transformation methods wherein the polynucleotide construct is integrated into the genome of the cell, transient transformation methods wherein the polynucleotide construct is not integrated into the genome of the cell and virus mediated methods. Said polynucleotides may be introduced into a cell by for example, recombinant viral vectors (e.g. retroviruses, adenoviruses), liposome and the like. For example, transient transformation methods include for example microinjection, electroporation or particle bombardment. Said polynucleotides may be included in vectors, more particularly plasmids or virus, in view of being expressed in cells.

### Engineered immune cells

The present invention employs a T cell comprising at least one CAR as described above. In particular, said T cell comprises exogenous polynucleotide sequence encoding CAR. Genetically modified T cells employed in accordance with the present invention are activated and proliferate independently of antigen binding mechanisms.

Said T cell refers to a cell of hematopoietic origin functionally involved in the initiation and/or execution of innate and/or adaptative immune response. Said T cell can be derived from a stem cell. The stem cells can be adult stem cells, non-human embryonic stem cells, more particularly non-human stem cells, cord blood stem cells, progenitor cells, bone marrow stem cells, induced pluripotent stem cells, or hematopoietic stem cells. Representative human cells are CD34+ cells. Said T cell can be a T-cell selected from the group consisting of inflammatory T-lymphocytes, cytotoxic T-lymphocytes, regulatory T-lymphocytes or helper T-lymphocytes. In another embodiment, said cell can be derived from the group consisting of CD4+ T-lymphocytes and CD8+ T-lymphocytes. Prior to expansion and genetic modification of the cells of the invention, a source of cells can be obtained from a subject through a variety of non-limiting methods. Cells can be obtained from a number of non-limiting sources, including peripheral blood mononuclear cells, bone marrow, lymph node tissue, cord blood, thymus tissue, tissue from a site of infection, ascites, pleural effusion, spleen tissue, and tumors. In certain embodiments of the present invention, any number of T cell lines available and known to those skilled in the art, may be used. In another embodiment, said T cell can be derived from a healthy donor, from a patient diagnosed with cancer or from a patient diagnosed with an infection. In another embodiment, said T cell is part of a mixed population of cells which present different phenotypic characteristics. Modified T cells resistant to an immunosuppressive treatment and susceptible to be obtained by the previous method are encompassed in the scope of the present invention.

In another embodiment, said T cell employed according to the present invention comprises a polynucleotide encoding the CAR.

### Activation and expansion of T cells

Whether prior to or after genetic modification of the T cells, even if the genetically modified immune cells of the present invention are activated and proliferate independently of antigen binding mechanisms, the immune cells, particularly T-cells of the present invention can be further activated and expanded generally using methods as described, for example, in U.S. Patents 6,352,694; 6,534,055; 6,905,680; 6,692,964; 5,858,358; 6,887,466; 6,905,681; 7,144,575; 7,067,318; 7,172,869; 7,232,566; 7,175,843; 5,883,223; 6,905,874; 6,797,514; 6,867,041; and U.S. Patent Application Publication No. 20060121005. T cells can be expanded *in vitro.*

Generally, the T cells employed in accordance with the invention are expanded by contact with an agent that stimulates a CD3 TCR complex and a co-stimulatory molecule on the surface of the T cells to create an activation signal for the T-cell.

For example, chemicals such as calcium ionophore A23187, phorbol 12-myristate 13-acetate (PMA), or mitogenic lectins like phytohemagglutinin (PHA) can be used to create an activation signal for the T-cell.

As non limiting examples, T cell populations may be stimulated *in vitro* such as by contact with an anti-CD3 antibody, or antigen-binding fragment thereof, or an anti-CD2 antibody immobilized on a surface, or by contact with a protein kinase C activator (e.g., bryostatin) in conjunction with a calcium ionophore. For co-stimulation of an accessory molecule on the surface of the T cells, a ligand that binds the accessory molecule is used. For example, a population of T cells can be contacted with an anti-CD3 antibody and an anti-CD28 antibody, under conditions appropriate for stimulating proliferation of the T cells. Conditions appropriate for T cell culture include an appropriate media (e.g., Minimal Essential Media or RPMI Media 1640 or, X-vivo 5, (Lonza)) that may contain factors necessary for proliferation and viability, including serum (e.g., fetal bovine or human serum), interleukin-2 (IL-2), insulin, IFN-g, 1L-4, 1L-7, GM-CSF, -10, - 2, 1L-15, TGFp, and TNF- or any other additives for the growth of cells known to the skilled artisan. Other additives for the growth of cells include, but are not limited to, surfactant, plasmanate, and reducing agents such as N-acetyl-cysteine and 2-mercaptoethanoi. Media can include RPMI 1640, A1M-V, DMEM, MEM, a-MEM, F-12, X-Vivo 1, and X-Vivo 20, Optimizer, with added amino acids, sodium pyruvate, and vitamins, either serum-free or supplemented with an appropriate amount of serum (or plasma) or a defined set of hormones, and/or an amount of cytokine(s) sufficient for the growth and expansion of T cells. Antibiotics, e.g., penicillin and streptomycin, are included only in experimental cultures, not in cultures of cells that are to be infused into a subject. The target cells are maintained under conditions necessary to support growth, for example, an appropriate temperature (e.g., 37° C) and atmosphere (e.g., air plus 5% C02). T cells that have been exposed to varied stimulation times may exhibit different characteristics

Alternatively, said cells can be expanded by co-culturing with tissue or cells.

### Therapeutic applications

In another aspect, the present invention provides an engineered T-cell expressing a CD19-specific chimeric antigen receptor (CAR), which comprises at least one extracellular ligand binding domain specific for CD19, a transmembrane domain, and at least one intracellular signaling domain; wherein said extracellular domain comprises a single chain FV fragment derived from the monoclonal antibody 4G7, specific for CD19, said single chain FV fragment comprising the variable fragment of the CD19 monoclonal antibody 4G7 immunoglobulin gamma 1 heavy chain of SEQ ID NO: 3 and the variable fragment of the CD19 monoclonal antibody 4G7 immunoglobulin kappa light chain of SEQ ID NO: 4 or SEQ ID NO: 5, for use in combination with the antibody alemtuzumab in a methods for treating cancer in a subject in need thereof, said method comprising the following steps:
(a) Administering to the subject the antibody alemtuzumab;
(b)Administrating to said subject the engineered T cell.

Said treatment can be ameliorating, curative or prophylactic. It may be either part of an autologous immunotherapy or part of an allogenic immunotherapy treatment. By autologous, it is meant that cells, cell line or population of cells used for treating patients are originating from said patient or from a Human Leucocyte Antigen (HLA) compatible donor. By allogeneic is meant that the cells or population of cells used for treating patients are not originating from said patient but from a donor.

Cancers that may be treated may comprise nonsolid tumors (such as hematological tumors, including but not limited to pre-B ALL (pedriatic indication), adult ALL, mantle cell lymphoma, diffuse large B-cell lymphoma and the like. Types of cancers to be treated with the CARs of the invention include, but are not limited to certain leukemia or lymphoid malignancies. Adult tumors/cancers and pediatric tumors/cancers are also included.

According to a preferred embodiment of the invention, said treatment can be administrated into patients undergoing an immunosuppressive treatment. Indeed, the present invention preferably relies on cells or population of cells, which have been made resistant to at least one immunosuppressive agent due to the inactivation of a gene encoding a receptor for such immunosuppressive agent. In this aspect, the immunosuppressive treatment should help the selection and expansion of the T-cells according to the invention within the patient.

The administration of the cells or population of cells according to the present invention may be carried out in any convenient manner, including by aerosol inhalation, injection, ingestion, transfusion, implantation or transplantation. The compositions described herein may be administered to a patient subcutaneously, intradermaliy, intratumorally, intranodally, intramedullary, intramuscularly, by intravenous or intralymphatic injection, or intraperitoneally. In one embodiment, the cell compositions of the present invention are preferably administered by intravenous injection.

The administration of the cells or population of cells can consist of the administration of 10⁴-10⁹ cells per kg body weight, preferably 10⁵ to 10⁶ cells/kg body weight including all integer values of cell numbers within those ranges. The cells or population of cells can be administrated in one or more doses. Said effective amount of cells can be administrated as a single dose. Said effective amount of cells can be administrated as more than one dose over a period time. Timing of administration is within the judgment of managing physician and depends on the clinical condition of the patient. The cells or population of cells may have been obtained from any source, such as a blood bank or a donor. While individual needs vary, determination of optimal ranges of effective amounts of a given cell type for a particular disease or conditions within the skill of the art. An effective amount means an amount which provides a therapeutic or prophylactic benefit. The dosage administrated will be dependent upon the age, health and weight of the recipient, kind of concurrent treatment, if any, frequency of treatment and the nature of the effect desired.

Said effective amount of cells or composition comprising those cells can be administrated parenterally. Said administration can be an intravenous administration. Said administration can be directly done by injection within a tumor.

### Other definitions

- Unless otherwise specified, "a," "an," "the," and "at least one" are used interchangeably and mean one or more than one.- Amino acid residues in a polypeptide sequence are designated herein according to the one-letter code, in which, for example, Q means Gln or Glutamine residue, R means Arg or Arginine residue and D means Asp or Aspartic acid residue.
- Amino acid substitution means the replacement of one amino acid residue with another, for instance the replacement of an Arginine residue with a Glutamine residue in a peptide sequence is an amino acid substitution.
- Nucleotides are designated as follows: one-letter code is used for designating the base of a nucleoside: a is adenine, t is thymine, c is cytosine, and g is guanine. For the degenerated nucleotides, r represents g or a (purine nucleotides), k represents g or t, s represents g or c, w represents a or t, m represents a or c, y represents t or c (pyrimidine nucleotides), d represents g, a or t, v represents g, a or c, b represents g, t or c, h represents a, t or c, and n represents g, a, t or c.
- "As used herein, "nucleic acid" or "polynucleotides" refers to nucleotides and/or polynucleotides, such as deoxyribonucleic acid (DNA) or ribonucleic acid (RNA), oligonucleotides, fragments generated by the polymerase chain reaction (PCR), and fragments generated by any of ligation, scission, endonuclease action, and exonuclease action. Nucleic acid molecules can be composed of monomers that are naturally-occurring nucleotides (such as DNA and RNA), or analogs of naturally-occurring nucleotides (e.g., enantiomeric forms of naturally-occurring nucleotides), or a combination of both. Modified nucleotides can have alterations in sugar moieties and/or in pyrimidine or purine base moieties. Sugar modifications include, for example, replacement of one or more hydroxyl groups with halogens, alkyl groups, amines, and azido groups, or sugars can be functionalized as ethers or esters. Moreover, the entire sugar moiety can be replaced with sterically and electronically similar structures, such as aza-sugars and carbocyclic sugar analogs. Examples of modifications in a base moiety include alkylated purines and pyrimidines, acylated purines or pyrimidines, or other well-known heterocyclic substitutes. Nucleic acid monomers can be linked by phosphodiester bonds or analogs of such linkages. Nucleic acids can be either single stranded or double stranded.
- By chimeric antigen receptor (CAR) is intended molecules that combine a binding domain against a component present on the target cell, for example an antibody-based specificity for a desired antigen (e.g., tumor antigen) with a T cell receptor-activating intracellular domain to generate a chimeric protein that exhibits a specific anti-target cellular immune activity. Generally, CAR consists of an extracellular single chain antibody (scFvFc) fused to the intracellular signaling domain of the T cell antigen receptor complex zeta chain (scFvFc:ζ) and have the ability, when expressed in T cells, to redirect antigen recognition based on the monoclonal antibody's specificity. One example of CAR used in the present invention is a CAR directing against CD19 antigen comprising the amino acid sequence of SEQ ID NO: 14.
- The term "endonuclease" refers to any wild-type or variant enzyme capable of catalyzing the hydrolysis (cleavage) of bonds between nucleic acids within a DNA or RNA molecule, preferably a DNA molecule. Endonucleases do not cleave the DNA or RNA molecule irrespective of its sequence, but recognize and cleave the DNA or RNA molecule at specific polynucleotide sequences, further referred to as "target sequences" or "target sites". Endonucleases can be classified as rare-cutting endonucleases when having typically a polynucleotide recognition site greater than 12 base pairs (bp) in length, more preferably of 14-55 bp. Rare-cutting endonucleases significantly increase HR by inducing DNA double-strand breaks (DSBs) at a defined locus (Perrin, Buckle et al. 1993; Rouet, Smih et al. 1994; Choulika, Perrin et al. 1995; Pingoud and Silva 2007). Rare-cutting endonucleases can for example be a homing endonuclease (Paques and Duchateau 2007), a chimeric Zinc-Finger nuclease (ZFN) resulting from the fusion of engineered zinc-finger domains with the catalytic domain of a restriction enzyme such as Fokl (Porteus and Carroll 2005), a Cas9 endonuclease from CRISPR system (Gasiunas, Barrangou et al. 2012; Jinek, Chylinski et al. 2012; Cong, Ran et al. 2013; Mali, Yang et al. 2013) or a chemical endonuclease (Eisenschmidt, Lanio et al. 2005; Arimondo, Thomas et al. 2006). In chemical endonucleases, a chemical or peptidic cleaver is conjugated either to a polymer of nucleic acids or to another DNA recognizing a specific target sequence, thereby targeting the cleavage activity to a specific sequence. Chemical endonucleases also encompass synthetic nucleases like conjugates of orthophenanthroline, a DNA cleaving molecule, and triplex-forming oligonucleotides (TFOs), known to bind specific DNA sequences (Kalish and Glazer 2005). Such chemical endonucleases are comprised in the term "endonuclease" according to the present invention.
- By a "TALE-nuclease" (TALEN) is intended a fusion protein consisting of a nucleic acid-binding domain typically derived from a Transcription Activator Like Effector (TALE) and one nuclease catalytic domain to cleave a nucleic acid target sequence. The catalytic domain is preferably a nuclease domain and more preferably a domain having endonuclease activity, like for instance I-Tevl, ColE7, NucA and Fok-I. In a particular embodiment, the TALE domain can be fused to a meganuclease like for instance I-Crel and I-Onul or functional variant thereof. In a more preferred embodiment, said nuclease is a monomeric TALE-Nuclease. A monomeric TALE-Nuclease is a TALE-Nuclease that does not require dimerization for specific recognition and cleavage, such as the fusions of engineered TAL repeats with the catalytic domain of I-Tevl described in WO2012138927. Transcription Activator like Effector (TALE) are proteins from the bacterial species *Xanthomonas* comprise a plurality of repeated sequences, each repeat comprising di-residues in position 12 and 13 (RVD) that are specific to each nucleotide base of the nucleic acid targeted sequence. Binding domains with similar modular base-per-base nucleic acid binding properties (MBBBD) can also be derived from new modular proteins recently discovered by the applicant in a different bacterial species. The new modular proteins have the advantage of displaying more sequence variability than TAL repeats. Preferably, RVDs associated with recognition of the different nucleotides are HD for recognizing C, NG for recognizing T, NI for recognizing A, NN for recognizing G or A, NS for recognizing A, C, G or T, HG for recognizing T, IG for recognizing T, NK for recognizing G, HA for recognizing C, ND for recognizing C, HI for recognizing C, HN for recognizing G, NA for recognizing G, SN for recognizing G or A and YG for recognizing T, TL for recognizing A, VT for recognizing A or G and SW for recognizing A. In another embodiment, critical amino acids 12 and 13 can be mutated towards other amino acid residues in order to modulate their specificity towards nucleotides A, T, C and G and in particular to enhance this specificity. TALE-nuclease have been already described and used to stimulate gene targeting and gene modifications (Boch, Scholze et al. 2009; Moscou and Bogdanove 2009; Christian, Cermak et al. 2010; Li, Huang et al. 2011). Engineered TAL-nucleases are commercially available under the trade name TALEN^{™} (Cellectis, 8 rue de la CroixJarry, 75013 Paris, France).

The rare-cutting endonuclease according to the present invention can also be a Cas9 endonuclease. Recently, a new genome engineering tool has been developed based on the RNA-guided Cas9 nuclease (Gasiunas, Barrangou et al. 2012; Jinek, Chylinski et al. 2012; Cong, Ran et al. 2013; Mali, Yang et al. 2013) from the type II prokaryotic CRISPR (Clustered Regularly Interspaced Short palindromic Repeats) adaptive immune system (see for review (Sorek, Lawrence et al. 2013)). The CRISPR Associated (Cas) system was first discovered in bacteria and functions as a defense against foreign DNA, either viral or plasmid. CRISPR-mediated genome engineering first proceeds by the selection of target sequence often flanked by a short sequence motif, referred as the proto-spacer adjacent motif (PAM). Following target sequence selection, a specific crRNA, complementary to this target sequence is engineered. Trans-activating crRNA (tracrRNA) required in the CRISPR type II systems paired to the crRNA and bound to the provided Cas9 protein. Cas9 acts as a molecular anchor facilitating the base pairing of tracRNA with cRNA (Deltcheva, Chylinski et al. 2011). In this ternary complex, the dual tracrRNA:crRNA structure acts as guide RNA that directs the endonuclease Cas9 to the cognate target sequence. Target recognition by the Cas9-tracrRNA:crRNA complex is initiated by scanning the target sequence for homology between the target sequence and the crRNA. In addition to the target sequence-crRNA complementarity, DNA targeting requires the presence of a short motif adjacent to the protospacer (protospacer adjacent motif - PAM). Following pairing between the dual-RNA and the target sequence, Cas9 subsequently introduces a blunt double strand break 3 bases upstream of the PAM motif (Garneau, Dupuis et al. 2010).

Rare-cutting endonuclease can be a homing endonuclease, also known under the name of meganuclease. Such homing endonucleases are well-known to the art (Stoddard 2005). Homing endonucleases recognize a DNA target sequence and generate a single- or double-strand break. Homing endonucleases are highly specific, recognizing DNA target sites ranging from 12 to 45 base pairs (bp) in length, usually ranging from 14 to 40 bp in length. The homing endonuclease according to the invention may for example correspond to a LAGLIDADG endonuclease, to a HNH endonuclease, or to a GIY-YIG endonuclease. Preferred homing endonuclease according to the present invention can be an I-*Crel* variant.
- By " delivery vector" or " delivery vectors" is intended any delivery vector which can be used in the present invention to put into cell contact ( i.e "contacting") or deliver inside cells or subcellular compartments (i.e "introducing") agents/chemicals and molecules (proteins or nucleic acids) needed in the present invention. It includes, but is not limited to liposomal delivery vectors, viral delivery vectors, drug delivery vectors, chemical carriers, polymeric carriers, lipoplexes, polyplexes, dendrimers, microbubbles (ultrasound contrast agents), nanoparticles, emulsions or other appropriate transfer vectors. These delivery vectors allow delivery of molecules, chemicals, macromolecules (genes, proteins), or other vectors such as plasmids, peptides developed by Diatos. In these cases, delivery vectors are molecule carriers. By "delivery vector" or "delivery vectors" is also intended delivery methods to perform transfection.
- The terms "vector" or "vectors" refer to a nucleic acid molecule capable of transporting another nucleic acid to which it has been linked. A "vector" in the present invention includes, but is not limited to, a viral vector, a plasmid, a RNA vector or a linear or circular DNA or RNA molecule which may consists of a chromosomal, non chromosomal, semi-synthetic or synthetic nucleic acids. Preferred vectors are those capable of autonomous replication (episomal vector) and/or expression of nucleic acids to which they are linked (expression vectors). Large numbers of suitable vectors are known to those of skill in the art and commercially available.

Viral vectors include retrovirus, adenovirus, parvovirus (e. g. adenoassociated viruses), coronavirus, negative strand RNA viruses such as orthomyxovirus (e. g., influenza virus), rhabdovirus (e. g., rabies and vesicular stomatitis virus), paramyxovirus (e. g. measles and Sendai), positive strand RNA viruses such as picornavirus and alphavirus, and double-stranded DNA viruses including adenovirus, herpesvirus (e. g., Herpes Simplex virus types 1 and 2, Epstein-Barr virus, cytomegalovirus), and poxvirus (e. g., vaccinia, fowlpox and canarypox). Other viruses include Norwalk virus, togavirus, flavivirus, reoviruses, papovavirus, hepadnavirus, and hepatitis virus, for example. Examples of retroviruses include: avian leukosis-sarcoma, mammalian C-type, B-type viruses, D type viruses, HTLV-BLV group, lentivirus, spumavirus (Coffin, J. M., Retroviridae: The viruses and their replication, In Fundamental Virology, Third Edition, B. N. Fields, et al., Eds., Lippincott-Raven Publishers, Philadelphia, 1996).
- By "lentiviral vector" is meant HIV-Based lentiviral vectors that are very promising for gene delivery because of their relatively large packaging capacity, reduced immunogenicity and their ability to stably transduce with high efficiency a large range of different cell types. Lentiviral vectors are usually generated following transient transfection of three (packaging, envelope and transfer) or more plasmids into producer cells. Like HIV, lentiviral vectors enter the target cell through the interaction of viral surface glycoproteins with receptors on the cell surface. On entry, the viral RNA undergoes reverse transcription, which is mediated by the viral reverse transcriptase complex. The product of reverse transcription is a double-stranded linear viral DNA, which is the substrate for viral integration in the DNA of infected cells. By "integrative lentiviral vectors (or LV)", is meant such vectors as nonlimiting example, that are able to integrate the genome of a target cell. At the opposite by "non-integrative lentiviral vectors (or NILV)" is meant efficient gene delivery vectors that do not integrate the genome of a target cell through the action of the virus integrase.
- Delivery vectors and vectors can be associated or combined with any cellular permeabilization techniques such as sonoporation or electroporation or derivatives of these techniques.
- By cell or cells is intended any eukaryotic living cells, primary cells and cell lines derived from these organisms for *in vitro* cultures.
- By "primary cell" or "primary cells" are intended cells taken directly from living tissue (i.e. biopsy material) and established for growth in vitro, that have undergone very few population doublings and are therefore more representative of the main functional components and characteristics of tissues from which they are derived from, in comparison to continuous tumorigenic or artificially immortalized cell lines.

As non limiting examples cell lines can be selected from the group consisting of CHO-K1 cells; HEK293 cells; Caco2 cells; U2-OS cells; NIH 3T3 cells; NSO cells; SP2 cells; CHO-S cells; DG44 cells; K-562 cells, U-937 cells; MRC5 cells; IMR90 cells; Jurkat cells; HepG2 cells; HeLa cells; HT-1080 cells; HCT-116 cells; Hu-h7 cells; Huvec cells; Molt 4 cells.

All these cell lines can be modified by the method of the present invention to provide cell line models to produce, express, quantify, detect, study a gene or a protein of interest; these models can also be used to screen biologically active molecules of interest in research and production and various fields such as chemical, biofuels, therapeutics and agronomy as non-limiting examples.
- by "mutation" is intended the substitution, deletion, insertion of up to one, two, three, four, five, six, seven, eight, nine, ten, eleven, twelve, thirteen, fourteen, fifteen, twenty, twenty five, thirty, fourty, fifty, or more nucleotides/amino acids in a polynucleotide (cDNA, gene) or a polypeptide sequence. The mutation can affect the coding sequence of a gene or its regulatory sequence. It may also affect the structure of the genomic sequence or the structure/stability of the encoded mRNA.
- by "variant(s)", it is intended a repeat variant, a variant, a DNA binding variant, a TALE-nuclease variant, a polypeptide variant obtained by mutation or replacement of at least one residue in the amino acid sequence of the parent molecule.
- by "functional variant" is intended a catalytically active mutant of a protein or a protein domain; such mutant may have the same activity compared to its parent protein or protein domain or additional properties, or higher or lower activity.
- "identity" refers to sequence identity between two nucleic acid molecules or polypeptides. Identity can be determined by comparing a position in each sequence which may be aligned for purposes of comparison. When a position in the compared sequence is occupied by the same base, then the molecules are identical at that position. A degree of similarity or identity between nucleic acid or amino acid sequences is a function of the number of identical or matching nucleotides at positions shared by the nucleic acid sequences. Various alignment algorithms and/or programs may be used to calculate the identity between two sequences, including FASTA, or BLAST which are available as a part of the GCG sequence analysis package (University of Wisconsin, Madison, Wis.), and can be used with, e.g., default setting. For example, polypeptides having at least 70%, 85%, 90%, 95%, 98% or 99% identity to specific polypeptides described herein and preferably exhibiting substantially the same functions, as well as polynucleotide encoding such polypeptides, are contemplated.
- "similarity" describes the relationship between the amino acid sequences of two or more polypeptides. BLASTP may also be used to identify an amino acid sequence having at least 70%, 75%, 80%, 85%, 87.5%, 90%, 92.5%, 95%, 97.5%, 98%, 99% sequence similarity to a reference amino acid sequence using a similarity matrix such as BLOSUM45, BLOSUM62 or BLOSUM80. Unless otherwise indicated a similarity score will be based on use of BLOSUM62. When BLASTP is used, the percent similarity is based on the BLASTP positives score and the percent sequence identity is based on the BLASTP identities score. BLASTP "Identities" shows the number and fraction of total residues in the high scoring sequence pairs which are identical; and BLASTP "Positives" shows the number and fraction of residues for which the alignment scores have positive values and which are similar to each other. Amino acid sequences having these degrees of identity or similarity or any intermediate degree of identity of similarity to the amino acid sequences disclosed herein are contemplated and encompassed by this disclosure. The polynucleotide sequences of similar polypeptides are deduced using the genetic code and may be obtained by conventional means. A polynucleotide encoding such a functional variant would be produced by reverse translating its amino acid sequence using the genetic code.
- "signal-transducing domain" or "co-stimulatory ligand" refers to a molecule on an antigen presenting cell that specifically binds a cognate co-stimulatory molecule on a T-cell, thereby providing a signal which, in addition to the primary signal provided by, for instance, binding of a TCR/CD3 complex with an MHC molecule loaded with peptide, mediates a T cell response, including, but not limited to, proliferation activation, differentiation and the like. A co-stimulatory ligand can include but is not limited to CD7, B7-1 (CD80), B7-2 (CD86), PD-L1, PD-L2, 4-1BBL, OX40L, inducible costimulatory igand (ICOS-L), intercellular adhesion molecule (ICAM, CD30L, CD40, CD70, CD83, HLA-G, MICA, M1CB, HVEM, lymphotoxin beta receptor, 3/TR6, ILT3, ILT4, an agonist or antibody that binds Toll ligand receptor and a ligand that specifically binds with B7-H3. A co-stimulatory ligand also encompasses, inter alia, an antibody that specifically binds with a co-stimulatory molecule present on a T cell, such as but not limited to, CD27, CD28, 4-IBB, OX40, CD30, CD40, PD-1, ICOS, lymphocyte function-associated antigen-1 (LFA-1), CD2, CD7, LTGHT, NKG2C, B7-H3, a ligand that specifically binds with CD83.

A "co-stimulatory molecule" refers to the cognate binding partner on a Tcell that specifically binds with a co-stimulatory ligand, thereby mediating a co-stimulatory response by the cell, such as, but not limited to proliferation. Co-stimulatory molecules include, but are not limited to an MHC class I molecule, BTLA and Toll ligand receptor.

A "co-stimulatory signal" as used herein refers to a signal, which in combination with primary signal, such as TCR/CD3 ligation, leads to T cell proliferation and/or upregulation or downregulation of key molecules.
- The term "extracellular ligand-binding domain" as used herein is defined as an oligo- or polypeptide that is capable of binding a ligand. Preferably, the domain will be capable of interacting with a cell surface molecule. For example, the extracellular ligand-binding domain may be chosen to recognize a ligand that acts as a cell surface marker on target cells associated with a particular disease state. Thus examples of cell surface markers that may act as ligands include those associated with viral, bacterial and parasitic infections, autoimmune disease and cancer cells.

The term "subject" or "patient" as used herein includes all members of the animal kingdom including non-human primates and humans.

The above written description of the invention provides a manner and process of making and using it such that any person skilled in this art is enabled to make and use the same, this enablement being provided in particular for the subject matter of the appended claims, which make up a part of the original description.

Where a numerical limit or range is stated herein, the endpoints are included. Also, all values and subranges within a numerical limit or range are specifically included as if explicitly written out.

Having generally described this invention, a further understanding can be obtained by reference to certain specific examples, which are provided herein for purposes of illustration only, and are not intended to be limiting unless otherwise specified.

### Examples

### Example 1: Proliferation of TCRalpha inactivated cells expressing a 4G7-CAR.

Heterodimeric TALE-nuclease targeting two 17-bp long sequences (called half targets) separated by an 15-bp spacer within T-cell receptor alpha constant chain region (TRAC) gene were designed and produced. Each half target is recognized by repeats of the half TALE-nucleases listed in Table 1.

| **Target** | **Target sequence** | **Repeat sequence** | **Half TALE-nuclease** |
|---|---|---|---|
| TRAC_T01 | TTGTCCCACAGATATCC Agaaccctgaccctg CCGTGTACCAGCTGAGA (SEQ ID NO: 20) | Repeat TRAC_T01-L (SEQ ID NO: 21) | TRAC_T01-L TALEN (SEQ ID NO: 23) |
| | | Repeat TRAC_T01-R (SEQ ID NO: 22) | TRAC_T01-R TALEN (SEQ ID NO: 24) |

Each TALE-nuclease construct was subcloned using restriction enzyme digestion in a mammalian expression vector under the control of the T7 promoter. mRNA encoding TALE-nuclease cleaving TRAC genomic sequence were synthesized from plasmid carrying the coding sequence downstream from the T7 promoter.

Purified T cells preactivated during 72 hours with antiCD3/CD28 coated beads were transfected with each of the 2 mRNAs encoding both half TRAC_T01 TALE-nucleases. 48 hours post-transfection, T cells were transduced with a lentiviral vector encoding 4G7-CAR (SEQ ID NO: 14). 2 days post-transduction, CD3_{NEG} cells were purified using anti-CD3 magnetic beads and 5 days post-transduction cells were reactivated with soluble anti-CD28 (5 µg/ml).

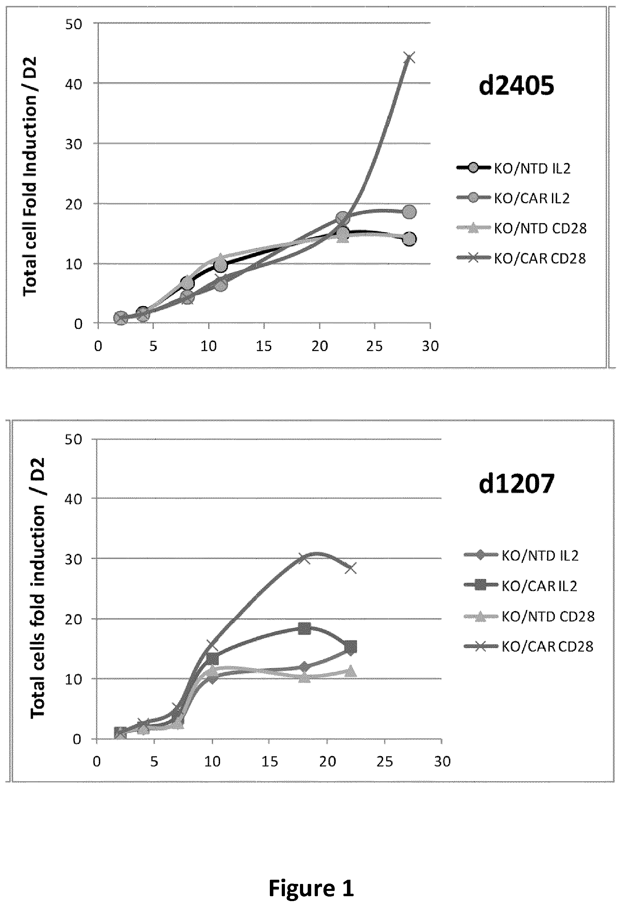

Cell proliferation was followed for up to 30 days after reactivation by counting cell 2 times per week. The Figure 1 shows the fold induction in cell number respect to the amount of cells present at day 2 post reactivation for two different donors. Increased proliferation in TCR alpha inactivated cells expressing the 4G7-CAR, especially when reactivated with anti-CD28, was observed compared to non transduced cells.

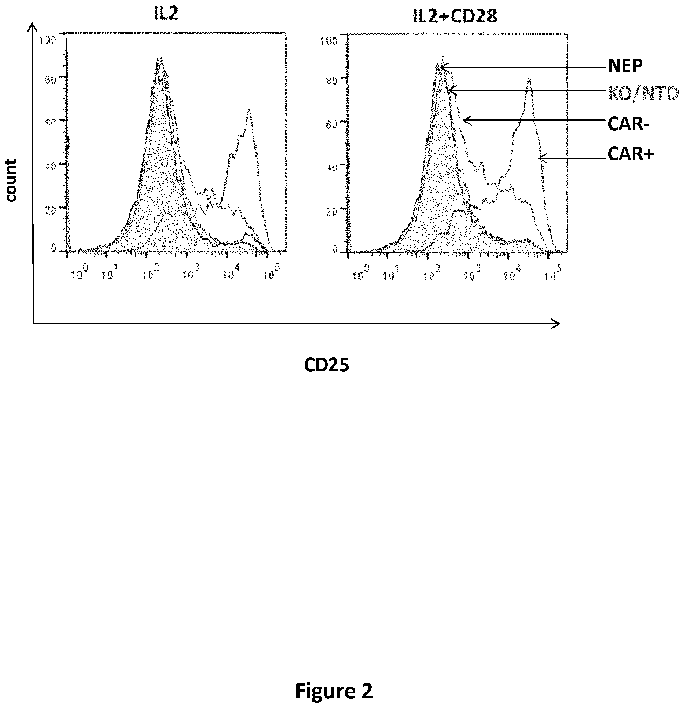

To investigate whether the human T cells expressing the 4G7-CAR display activated state, the expression of the activation marker CD25 was analyzed by FACS 7 days post transduction. As indicated in Figure 2, purified cells transduced with the lentiviral vector encoding 4G7-CAR expressed considerably more CD25 at their surface than the non transduced cells. Increased CD25 expression is observed both in CD28 reactivation or no reactivation conditions.

### Example 2: Comparison of basal activation of primary human T cells expressing the 4G7-CAR and the classical FMC63-CAR.

To determine whether 4G7 scFV confers a prolonged "activated" state on the transduced cell, basal activation of T cell transduced with CAR harboring a 4G7 scFV (SEQ ID NO: 17 encoded SEQ ID NO: 15) or a classical FMC63 scFV (SEQ ID NO: 16) was compared.

Purified human T cells were transduced according to the following protocol: briefly, 1×10⁶ CD3+ cells preactivated during 3 days with anti CD3/CD28 coated beads and recombinant IL2 were transduced with lentiviral vectors encoding the 4G7-CAR (SEQ ID NO: 15) and the FMC63-CAR (SEQ ID NO: 16) at an MOI of 5 in 12-well non tissue culture plates coated with 30µg/ml retronectin. 24 hours post transduction the medium was removed and replaced by fresh medium. The cells were then maintained at a concentration of 1×10⁶ cells/ml throughout the culture period by cell enumeration every 2-3 days.

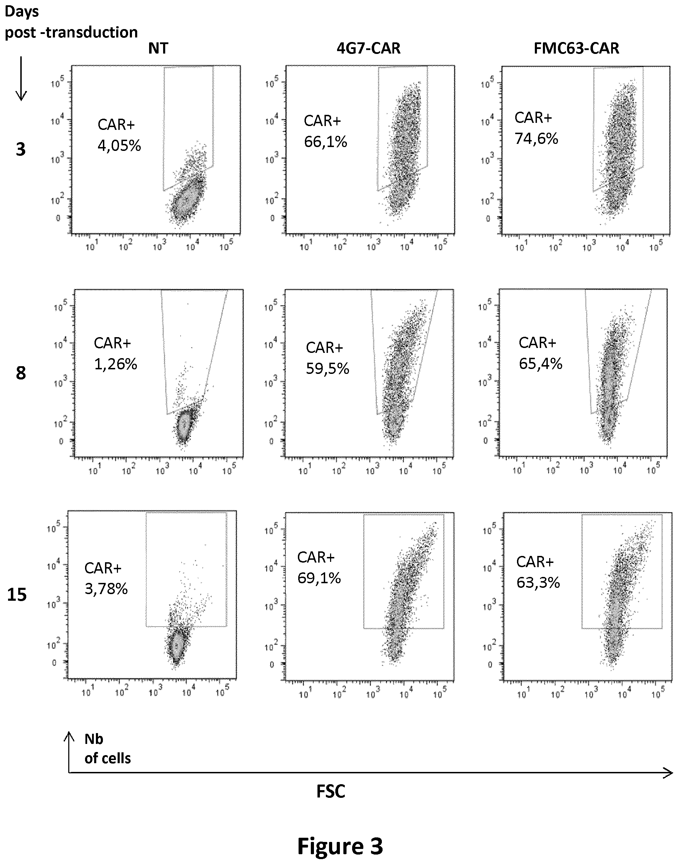

3, 8 and 15 days post transduction with the lentiviral vector encoding either the 4G7-CAR or the FMC63-CAR, the percentage of CAR expressing cells was assessed by flow cytometry. It was observed that the efficiency of transduction was relatively equivalent with the two lentiviral vectors Figure 3.

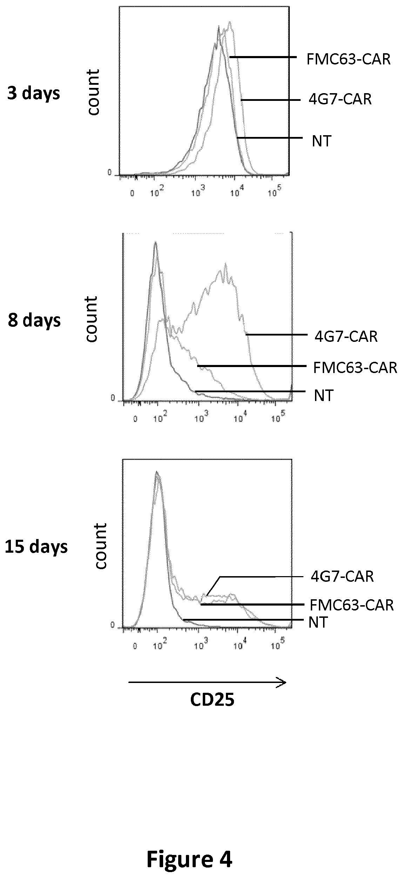

It was then investigated whether the human T cells expressing the 4G7-CAR exhibited a more activated state than the human T cells expressing the FMC63-CAR. For that purpose the expression of the activation marker CD25 was compared at the surface of T cells transduced with the 2 lentiviral vectors at different time points. As indicated in the Figure 4, 3 and 8 days post transduction, the cells transduced with the lentiviral vector encoding the 4G7-CAR expressed considerably more CD25 at their surface than the cells transduced with the lentiviral vector encoding the FMC63-CAR.

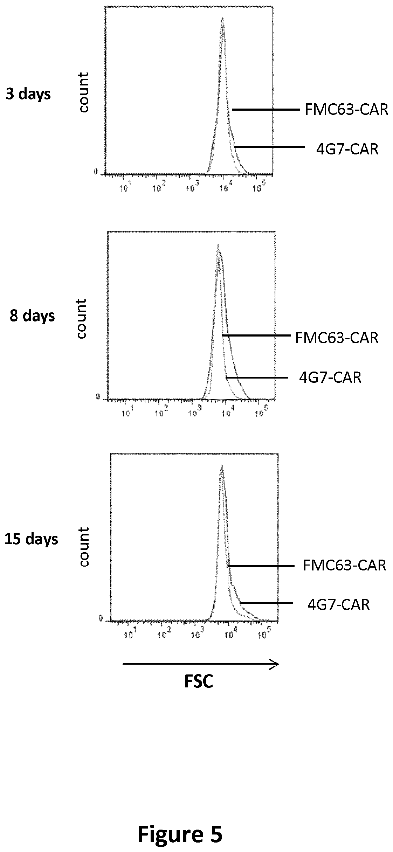

The size of the 4G7-CAR or FMC63-CAR transduced cells was also assessed by flow cytometry at different time points. It was observed that the cells expressing the 4G7-CAR were bigger than the cells expressing the FMC63-CAR 3, 8 and 15 days post transduction Figure 5.

Following non-specific activation in vitro, 4G7-CAR transduced cells display an increased cell size (blast formation) as well as the expression of activation markers (CD25) over an extended time period. This long-term activation permits extended proliferation compared to cells transduced with a similar CAR containing the FMC63 ScFv.

### Example 3: Comparison of proliferation of primary human T cells expressing the 4G7-CAR and the classical FMC63-CAR.

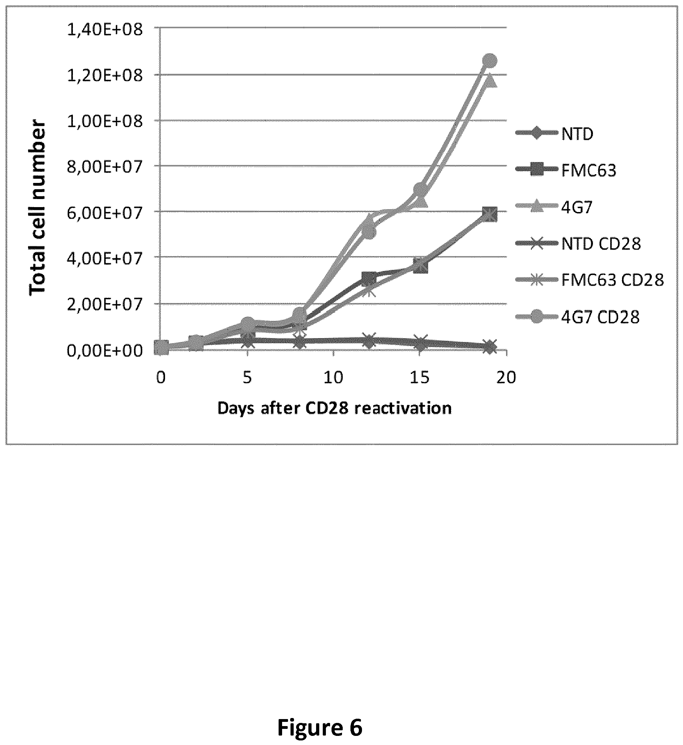

To determine whether 4G7 scFV confers a higher proliferation activity, proliferation of T cell transduced with CAR harboring a 4G7 scFV (SEQ ID NO: 17 encoded SEQ ID NO: 15) or a classical FMC63 scFV (SEQ ID NO: 16) was followed up to 20 days by counting cell two times per week. Purified human T cells were transduced according to the following protocol: briefly, 1×10⁶ CD3+ cells preactivated during 3 days with anti CD3/CD28 coated beads and recombinant IL2 were transduced with lentiviral vectors encoding the 4G7-CAR (SEQ ID NO: 15) and the FMC63-CAR (SEQ ID NO: 16). The cells were then maintained under classical conditions and were reactivated at Day 12. Cells were seeded at the same density and were counted two times per week during 20 days. As represented in figure 6, proliferation activity of T-cells expressing the 4G7-CAR is twofold higher compared to those of cells expressing the classical FMC63-CAR.

### REFERENCES

Arimondo, P. B., C. J. Thomas, et al. (2006). "Exploring the cellular activity of camptothecin-triple-helix-forming oligonucleotide conjugates." Mol Cell Biol 26(1): 324-33.
Atkins, J. F., N. M. Wills, et al. (2007). "A case for "StopGo": reprogramming translation to augment codon meaning of GGN by promoting unconventional termination (Stop) after addition of glycine and then allowing continued translation (Go)." Rna 13(6): 803-10.
Bierer, B. E., G. Hollander, et al. (1993). "Cyclosporin A and FK506: molecular mechanisms of immunosuppression and probes for transplantation biology." Curr Opin Immunol 5(5): 763-73.
Boch, J., H. Scho!ze, et al. (2009). "Breaking the code of DNA binding specificity of TAL-type III effectors." Science 326(5959): 1509-12.
Choulika, A., A. Perrin, et al. (1995). "Induction of homologous recombination in mammalian chromosomes by using the I-Scel system of Saccharomyces cerevisiae." Mol Cell Biol 15(4): 1968-73.
Christian, M., T. Cermak, et al. (2010). "Targeting DNA double-strand breaks with TAL effector nucleases." Genetics 186(2): 757-61.
Cong, L., F. A. Ran, et al. (2013). "Multiplex genome engineering using CRISPR/Cas systems." Science 339(6121): 819-23.
Deltcheva, E., K. Chylinski, et al. (2011). "CRISPR RNA maturation by trans-encoded small RNA and host factor RNase III." Nature 471(7340): 602-7.
Donnelly, M. and G. Elliott (2001). "Nuclear localization and shuttling of herpes simplex virus tegument protein VP13/14." J Virol 75(6): 2566-74.
Doronina, V. A., C. Wu, et al. (2008). "Site-specific release of nascent chains from ribosomes at a sense codon." Mol Cell Biol 28(13): 4227-39.
Eisenschmidt, K., T. Lanio, et al. (2005). "Developing a programmed restriction endonuclease for highly specific DNA cleavage." Nucleic Acids Res 33(22): 7039-47.
Garneau, J. E., M. E. Dupuis, et al. (2010). "The CRISPR/Cas bacterial immune system cleaves bacteriophage and plasmid DNA." Nature 468(7320): 67-71.
Gasiunas, G., R. Barrangou, et al. (2012). "Cas9-crRNA ribonucleoprotein complex mediates specific DNA cleavage for adaptive immunity in bacteria." Proc Natl Acad Sci U S A 109(39): E2579-86.
Henderson, D. J., I. Naya, et al. (1991). "Comparison of the effects of FK-506, cyclosporin A and rapamycin on IL-2 production." Immunology 73(3): 316-21.
Jena, B., G. Dotti, et al. (2010). "Redirecting T-cell specificity by introducing a tumor-specific chimeric antigen receptor." Blood 116(7): 1035-44.
Jinek, M., K. Chylinski, et al. (2012). "A programmable dual-RNA-guided DNA endonuclease in adaptive bacterial immunity." Science 337(6096): 816-21.
Kalish, J. M. and P. M. Glazer (2005). "Targeted genome modification via triple helix formation." Ann N Y Acad Sci 1058: 151-61.
Li, T., S. Huang, et al. (2011). "TAL nucleases (TALNs): hybrid proteins composed of TAL effectors and Fokl DNA-cleavage domain." Nucleic Acids Res 39(1): 359-72.
Liu, J., M. W. Albers, et al. (1992). "Inhibition of T cell signaling by immunophilin-ligand complexes correlates with loss of calcineurin phosphatase activity." Biochemistry 31(16): 3896-901.
Mali, P., L. Yang, et al. (2013). "RNA-guided human genome engineering via Cas9." Science 339(6121): 823-6.
Moscou, M. J. and A. J. Bogdanove (2009). "A simple cipher governs DNA recognition by TAL effectors." Science 326(5959): 1501.
Paques, F. and P. Duchateau (2007). "Meganucleases and DNA double-strand break-induced recombination: perspectives for gene therapy." Curr Gene Ther 7(1): 49-66.
Park, T. S., S. A. Rosenberg, et al. (2011). "Treating cancer with genetically engineered T cells." Trends Biotechnol 29(11): 550-7.
Peipp, M., D. Saul, et al. (2004). "Efficient eukaryotic expression of fluorescent scFv fusion proteins directed against CD antigens for FACS applications." J Immunol Methods 285(2): 265-80.
Perrin, A., M. Buckle, et al. (1993). "Asymmetrical recognition and activity of the I-Scel endonuclease on its site and on intron-exon junctions." Embo J 12(7): 2939-47.
Pingoud, A. and G. H. Silva (2007). "Precision genome surgery." Nat Biotechnol 25(7): 743-4.
Porteus, M. H. and D. Carroll (2005). "Gene targeting using zinc finger nucleases." Nat Biotechnol 23(8): 967-73.
Rouet, P., F. Smih, et al. (1994). "Introduction of double-strand breaks into the genome of mouse cells by expression of a rare-cutting endonuclease." Mol Cell Biol 14(12): 8096-106.
Sorek, R., C. M. Lawrence, et al. (2013). "CRISPR-mediated Adaptive Immune Systems in Bacteria and Archaea." Annu Rev Biochem.
Stoddard, B. L. (2005). "Homing endonuclease structure and function." Q Rev Biophys 38(1): 49-95.

## Claims

1. A composition for use in combination therapy comprising:
(a) an engineered T-cell expressing a CD19-specific chimeric antigen receptor (CAR), which comprises at least one extracellular ligand binding domain specific for CD19, a transmembrane domain, and at least one intracellular signaling domain; wherein said extracellular domain comprises a single chain FV fragment derived from the monoclonal antibody 4G7, specific for CD19, said single chain FV fragment comprising the variable fragment of the CD19 monoclonal antibody 4G7 immunoglobulin gamma 1 heavy chain of SEQ ID NO: 3 and the variable fragment of the CD19 monoclonal antibody 4G7 immunoglobulin kappa light chain of SEQ ID NO: 4 or SEQ ID NO: 5; and
(b) the antibody alemtuzumab.

2. The composition for use according to claim 1, wherein said single chain FV fragment comprises the amino acid sequence of SEQ ID NOs: 7 or 8.

3. The composition for use according to claim1 or 2, wherein the at least one intracellular signaling domain of the CD19-specific CAR is a CD3 zeta signaling domain comprising the amino acid sequence of SEQ ID NO: 10.

4. The composition for use according to any one of claims 1 to 3, wherein the transmembrane domain of the CD19-specific CAR comprises a human CD8 alpha chain transmembrane and stalk domain comprising the amino acid sequence of SEQ ID NO: 13.

5. The composition for use according to any one of claims 1 to 4, wherein the CD19-specific CAR comprises a second intracellular signaling domain.

6. The composition for use according to claim 5, wherein the second intracellular signaling domain comprises the amino acid sequence of SEQ ID NO: 11.

7. The composition for use according to claim 1, wherein the CD19-specific CAR comprises the amino acid sequence of SEQ ID NO: 14 or 15.

8. The composition for use according to any one of claims 1 to 7, wherein the engineered T-cell has been genetically modified by inactivating a gene encoding TCRalpha, TCRbeta, CD52, GR, PD1 or CTLA-4.

9. An engineered T-cell expressing a CD19-specific chimeric antigen receptor (CAR), which comprises at least one extracellular ligand binding domain specific for CD19, a transmembrane domain, and at least one intracellular signaling domain; wherein said extracellular domain comprises a single chain FV fragment derived from the monoclonal antibody 4G7, specific for CD19, said single chain FV fragment comprising the variable fragment of the CD19 monoclonal antibody 4G7 immunoglobulin gamma 1 heavy chain of SEQ ID NO: 3 and the variable fragment of the CD19 monoclonal antibody 4G7 immunoglobulin kappa light chain of SEQ ID NO: 4 or SEQ ID NO: 5, for use in combination with the antibody alemtuzumab in a method of treating cancer in a subject in need thereof, the method comprising:
administering to the subject the antibody alemtuzumab; and
administering to the subject the engineered T-cell.

10. The engineered T-cell for use of claim 9, wherein said single chain FV fragment comprises the amino acid sequence of SEQ ID NOs: 7 or 8.

11. The engineered T-cell for use of claim 9 or 10, wherein the at least one intracellular signaling domain of the CD19-specific CAR is a CD3 zeta signaling domain comprising the amino acid sequence of SEQ ID NO: 10.

12. The engineered T-cell for use of any one of claims 9 to 11, wherein the transmembrane domain of the CD19-specific CAR comprises a human CD8 alpha chain transmembrane and stalk domain comprising the amino acid sequence of SEQ ID NO: 13.

13. The engineered T-cell for use of any one of claims 9 to 12, wherein the CD19-specific CAR comprises a second intracellular signaling domain.

14. The engineered T-cell for use of claim 13, wherein the second intracellular signaling domain comprises the amino acid sequence of SEQ ID NO: 11.

15. The engineered T-cell for use of claim 9, wherein the CD19-specific CAR comprises the amino acid sequence of SEQ ID NO: 14 or 15.

16. The engineered T-cell for use of any one of claims 9 to 15, wherein the engineered T-cell has been genetically modified by inactivating a gene encoding TCRalpha, TCRbeta, CD52, GR, PD1 or CTLA-4.

## Patentansprüche

1. Zusammensetzung zur Verwendung in Kombinationstherapie, umfassend:
(a) eine veränderte T-Zelle, die einen chimären CD19-spezifischen Antigenrezeptor (CAR) exprimiert, der mindestens eine extrazellulären Ligandenbindungsdomäne spezifisch für CD19, eine Transmembrandomäne und mindestens eine intrazelluläre Signaldomäne umfasst; wobei die extrazelluläre Domäne ein einkettiges FV-Fragment umfasst, das von dem monoklonalen Antikörper 4G7, spezifisch für CD19, abgeleitet ist, wobei das einkettige FV-Fragment das variable Fragment der schweren Kette des monoklonalen CD19-Antikörpers 4G7 Immunglobulin Gamma 1 von SEQ. ID NO: 3 und das variable Fragment der leichten Kette des monoklonalen CD19-Antikörpers 4G7 Immunglobulin Kappa von SEQ. ID NO: 4 oder SEQ ID NO: 5 umfasst; und
(b) den Antikörper Alemtuzumab.

2. Zusammensetzung zur Verwendung nach Anspruch 1, wobei das einkettige FV-Fragment die Aminosäuresequenz von SEQ ID NO: 7 oder 8 umfasst.

3. Zusammensetzung zur Verwendung nach Anspruch 1 oder 2, wobei die mindestens eine intrazelluläre Signaldomäne des CD19-spezifischen CAR eine CD3-zeta-Signaldomäne, die die Aminosäuresequenz von SEQ ID NO: 10 umfasst, ist.

4. Zusammensetzung zur Verwendung nach einem der Ansprüche 1 bis 3, wobei die Transmembrandomäne des CD19-spezifischen CAR eine menschliche CD8-Alphakettentransmembran und -Kopfdomäne umfasst, die die Aminosäurensequenz von SEQ ID NO: 13 umfasst.

5. Zusammensetzung zur Verwendung nach einem der Ansprüche 1 bis 4, wobei das CD19-spezifische CAR eine zweite intrazelluläre Signaldomäne umfasst.

6. Zusammensetzung zur Verwendung nach Ansprüche 5, wobei die zweite intrazelluläre Signaldomäne die Aminosäurensequenz von SEQ ID NO: 11 umfasst.

7. Zusammensetzung zur Verwendung nach Anspruch 1, wobei das CD19-spezifische CAR die Aminosäurensequenz von SEQ ID NO: 14 oder 15 umfasst.

8. Zusammensetzung zur Verwendung nach einem der Ansprüche 1 bis 7, wobei die veränderte T-Zelle durch Inaktivieren eines Gens, das für TCRalpha, TCRbeta, CD52, GR, PD1 oder CTLA-4 codiert, genetisch modifiziert wurde.

9. Veränderte T-Zelle, die einen chimären CD19-spezifischen Antigenrezeptor (CAR) exprimiert, der mindestens eine extrazelluläre Ligandenbindungsdomäne spezifisch für CD19, eine Transmembrandomäne und mindestens eine intrazelluläre Signaldomäne umfasst; wobei die extrazelluläre Domäne ein einkettiges FV-Fragment, das von dem monoklonalen Antikörper 4G7, spezifisch für CD19, abgeleitet ist, umfasst, wobei das einkettige FV-Fragment das variable Fragment der schweren Kette des monoklonalen CD19-Antikörpers 4G7 Immunglobulin Gamma 1 von SEQ. ID NO: 3 und das variable Fragment der leichten Kette des monoklonalen CD19-Antikörpers 4G7 Immunglobulin Kappa von SEQ. ID NO: 4 oder SEQ ID NO: 5 umfasst, zur Verwendung in Kombination mit dem Antikörper Alemtuzumab in einem Verfahren zum Behandeln von Krebs in einem dies benötigenden Subjekt, wobei das Verfahren Folgendes umfasst:
Verabreichen, an das Subjekt, des Antikörpers Alemtuzumab;
und
Verabreichen, an das Subjekt, der veränderten T-Zelle.

10. Veränderte T-Zelle zur Verwendung nach Anspruch 9, wobei das einkettige FV-Fragment die Aminosäuresequenz von SEQ ID NO: 7 oder 8 umfasst.

11. Veränderte T-Zelle zur Verwendung nach Anspruch 9 oder 10, wobei die mindestens eine intrazelluläre Signaldomäne des CD19-spezifischen CAR eine CD3-zeta-Signaldomäne, die die Aminosäuresequenz von SEQ ID NO: 10 umfasst, ist.

12. Veränderte T-Zelle zur Verwendung nach einem der Ansprüche 9 bis 11, wobei die Transmembrandomäne des CD19-spezifischen CAR eine menschliche CD8-Alphakettentransmembran und -Kopfdomäne umfasst, die die Aminosäurensequenz von SEQ ID NO: 13 umfasst.

13. Veränderte T-Zelle zur Verwendung nach einem der Ansprüche 9 bis 12, wobei das CD19-spezifische CAR eine zweite intrazelluläre Signaldomäne umfasst.

14. Veränderte T-Zelle zur Verwendung nach Anspruch 13, wobei die zweite intrazelluläre Signaldomäne die Aminosäurensequenz von SEQ ID NO: 11 umfasst.

15. Veränderte T-Zelle zur Verwendung nach Anspruch 9, wobei das CD19-spezifische CAR die Aminosäurensequenz von SEQ ID NO: 14 oder 15 umfasst.

16. Veränderte T-Zelle zur Verwendung nach einem der Ansprüche 9 bis 15, wobei die veränderte T-Zelle durch Inaktivieren eines Gens, das für TCRalpha, TCRbeta, CD52, GR, PD1 und CTLA-4 codiert, genetisch modifiziert wurde.

## Revendications

1. Composition destinée à être utilisée en thérapie combinée comprenant :
(a) un lymphocyte T modifié exprimant un récepteur de l'antigène chimère (CAR) spécifique de CD19, qui comprend au moins un domaine de liaison de ligand extracellulaire spécifique de CD19, un domaine transmembranaire et au moins un domaine de signalisation intracellulaire ; dans laquelle ledit domaine extracellulaire comprend un fragment FV à chaîne unique dérivé de l'anticorps monoclonal 4G7, spécifique de CD19, ledit fragment FV à chaîne unique comprenant le fragment variable de la chaîne lourde d'immunoglobuline gamma 1 de l'anticorps monoclonal 4G7 de CD19 de SEQ ID NO : 3 et le fragment variable de la chaîne légère d'immunoglobuline kappa de l'anticorps monoclonal 4G7 de CD19 de SEQ ID NO : 4 ou de SEQ ID NO : 5 ; et
(b) l'anticorps alemtuzumab.

2. Composition destinée à être utilisée selon la revendication 1, dans laquelle ledit fragment FV à chaîne unique comprend la séquence d'acides aminés de SEQ ID NO : 7 ou 8.

3. Composition destinée à être utilisée selon la revendication 1 ou 2, dans laquelle l'au moins un domaine de signalisation intracellulaire du CAR spécifique de CD19 est un domaine de signalisation zêta de CD3 comprenant la séquence d'acides aminés de SEQ ID NO : 10.

4. Composition destinée à être utilisée selon l'une quelconque des revendications 1 à 3, dans laquelle le domaine transmembranaire du CAR spécifique de CD19 comprend un domaine transmembranaire et tige de chaîne alpha de CD8 humain comprenant la séquence d'acides aminés de SEQ ID NO : 13.

5. Composition destinée à être utilisée selon l'une quelconque des revendications 1 à 4, dans laquelle le CAR spécifique de CD19 comprend un second domaine de signalisation intracellulaire.

6. Composition destinée à être utilisée selon la revendication 5, dans laquelle le second domaine de signalisation intracellulaire comprend la séquence d'acides aminés de SEQ ID NO : 11.

7. Composition destinée à être utilisée selon la revendication 1, dans laquelle le CAR spécifique de CD19 comprend la séquence d'acides aminés de SEQ ID NO : 14 ou 15.

8. Composition destinée à être utilisée selon l'une quelconque des revendications 1 à 7, dans laquelle le lymphocyte T modifié a été génétiquement modifié en inactivant un gène codant pour TCRalpha, TCRbêta, CD52, GR, PD1 ou CTLA-4.

9. Lymphocyte T modifié exprimant un récepteur de l'antigène chimère (CAR) spécifique de CD19, qui comprend au moins un domaine de liaison de ligand extracellulaire spécifique de CD19, un domaine transmembranaire et au moins un domaine de signalisation intracellulaire ; dans lequel ledit domaine extracellulaire comprend un fragment FV à chaîne unique dérivé de l'anticorps monoclonal 4G7, spécifique de CD19, ledit fragment FV à chaîne unique comprenant le fragment variable de la chaîne lourde d'immunoglobuline gamma 1 de l'anticorps monoclonal 4G7 de CD19 de SEQ ID NO : 3 et le fragment variable de la chaîne légère d'immunoglobuline kappa de l'anticorps monoclonal 4G7 de CD19 de SEQ ID NO : 4 ou de SEQ ID NO : 5, destiné à être utilisé en combinaison avec l'anticorps alemtuzumab dans un procédé de traitement du cancer chez un sujet en ayant besoin, le procédé comprenant :
l'administration au sujet de l'anticorps alemtuzumab ; et
l'administration au sujet du lymphocyte T modifié.

10. Lymphocyte T modifié destiné à être utilisé selon la revendication 9, dans lequel ledit fragment FV à chaîne unique comprend la séquence d'acides aminés de SEQ ID NO : 7 ou 8.

11. Lymphocyte T modifié destiné à être utilisé selon la revendication 9 ou 10, dans lequel l'au moins un domaine de signalisation intracellulaire du CAR spécifique de CD19 est un domaine de signalisation zêta de CD3 comprenant la séquence d'acides aminés de SEQ ID NO : 10.

12. Lymphocyte T modifié destiné à être utilisé selon l'une quelconque des revendications 9 à 11, dans lequel le domaine transmembranaire du CAR spécifique de CD19 comprend un domaine transmembranaire et tige de chaîne alpha de CD8 humain comprenant la séquence d'acides aminés de SEQ ID NO : 13.

13. Lymphocyte T modifié destiné à être utilisé selon l'une quelconque des revendications 9 à 12, dans lequel le CAR spécifique de CD19 comprend un second domaine de signalisation intracellulaire.

14. Lymphocyte T modifié destiné à être utilisé selon la revendication 13, dans lequel le second domaine de signalisation intracellulaire comprend la séquence d'acides aminés de SEQ ID NO : 11.

15. Lymphocyte T modifié destiné à être utilisé selon la revendication 9, dans lequel le CAR spécifique de CD19 comprend la séquence d'acides aminés de SEQ ID NO : 14 ou 15.

16. Lymphocyte T modifié destiné à être utilisé selon l'une quelconque des revendications 9 à 15, dans lequel le lymphocyte T modifié a été génétiquement modifié en inactivant un gène codant pour TCRalpha, TCRbêta, CD52, GR, PD1 ou CTLA-4.
